# EUROPEAN PATENT SPECIFICATION

(11) **EP 1 207 849 B1**
(45) Date of publication and mention of the grant of the patent: **05.01.2005**
(21) Application number: 99939602.1
(22) Date of filing: 06.08.1999
(51) Int. Cl.: A61K 7/48

(54) **USE OF N,N'-BIS(2-HYDROXYETHYL)NONANDIAMIDE (ALDEMIDROL) AS A COSMETIC AGENT**
VERWENDUNG VON N,N'-BIS(2-HYDROXYETHYL)-NONANDIAMID (ALDEMIDROL) ALS KOSMETISCHES MITTEL
UTILISATION DE N, N'-BIS(2-HYDROXYETHYL)-NONANEDIAMIDE (ALDEMIDROL) COMME AGENT COSMETIQUE

(43) Date of publication of application: 29.05.2002
(73) Proprietor: Innovet Italia S.r.l., 20144 Milano (IT)
(72) Inventor: COMELLI, Cristina, I-35141 Padova (IT); DELLA VALLE, Maria, Federica, I-35141 Padova (IT); DELLA VALLE, Francesco, I-35122 Padova (IT); MARCOLONGO, Gabriele, I-35020 Due Carrare-Padova (IT)
(74) Representative: Long, Giorgio
(86) International application number: PCT/IT1999/000258
(87) International publication number: WO 2001/010402

(56) References cited:
- WO-A-96/39119
- WO-A-98/20834
- WO-A-99/17714
- US-A- 3 875 301
- US-A- 5 618 842
- US-A- 5 693 623
- CHEMICAL ABSTRACTS, vol. 86, no. 4, 24 January 1977 (1977-01-24) Columbus, Ohio, US; abstract no. 21750k, T. REBAGAY ET AL : "Correlation of dielectric constant and solubilizing properties of tetramethyldicarboxamides" page 335; XP002136658 & J. PHARM. SCI., vol. 65, no. 11, 1976, pages 1645-1648,

## Description

The present invention relates to the use of N,N¹-bis(2-hydroxyethyl)nonandiamide, the common international name of which is adelmidrol, for the preparation of cosmetic compositions for use on skin and/or mucous membranes which are irritable and/or are subject to acute irritation, in man and in animals.

The term "cosmetic products" means all preparations which can protect or keep in good condition the external surfaces of the human or animal body such as the epidermis, the piliferous system and the hair, the nails, the lips, the external genital organs, and the mucous membranes of the mouth.

The basic role of the skin and of the mucous membranes is to protect the underlying tissues from potentially harmful environmental agents and to prevent excessive loss of fluids from the body. The barrier function of the tegumentary tissues is due mainly to the proteinaceous and, above all, to the lipid characteristics of their multi-layered epithelia (Gniadecha M. et al., 1998, J. Inv. Dermatol. 110: 393-398). The most superficial epidermal and/or epithelial layers in fact protect against loss of water and provide an effective barrier which resists the entry of microorganisms (Elias P.M., 1988. Drug. Dev. Res. 13: 97-105).

It is known that this barrier function depends to a large extent on the proliferative and differentiational processes of the epidermal keratinocytes of the skin and of the epithelial cells of the mucous membranes (Brod J., 1991, Int. J. Dermatol., 30: 84-90).

It is known that the hydration state of the skin is a parameter which can easily be measured by non-invasive methods applied to the skin surface (Thune P., 1989, Acta Derm. Venereol., suppl. 144: 133-135). The quantitative measurement of the hydration state of the skin utilizes basically two measurements: transepidermal evaporation of water (TEWL) and skin capacitance.

TEWL, which is measured by an instrument known as an atmometer, expresses, in g/m²/hour, the gradient of diffusion of water vapour through the epidermal tissue, given the known tendency of this gas to diffuse in accordance with the concentration gradient (Pinnagoda J. et al., 1990, Contact Dermatitis, 22: 164-178). The water content of the *stratus corneum*, on the other hand, is expressed by electrical capacitance measured by the application of a corneometer which records the water content on the surface of the skin, since water has the highest dielectric constant of all the skin components. An increase in skin water content brings about an increase in capacitance values which are expressed in arbitrary units by the corneometer (Werner Y., 1986, Acta Derm. Venereol., 66: 281-284).

It is known that the determination of the skin hydration level by measurement of the TEWL enables subjects with skin which is normally reactive to very diverse stimuli to be distinguished from subjects characterized by an irritable skin (Tupker R.A., 1990, Br. J. Dermatol., 123: 199-205).

It has been shown that the skin of atopic subjects is irritable in response to exogenous stimuli applied at skin level; in these subjects, the skin tends to appear dry (Tupker R.A. et al. 1990, Br. J. Dermatol., 123: 199-205). These characteristic behaviours correspond to an altered barrier function and to an increase in TEWL values (Seidenari S. et al., 1995, Acta Derm. Venereol., 75:429-433; Gollhausen R., 1991, in Ruzicka T. et al., eds., Handbook of Atopic Eczema, Springer Verlag, pp. 306-318; Thune P., 1989, Acta Derm. Venereol., suppl. 144: 133-135; Werner Y. et al., 1985, Acta Derm. Venereol., 65: 102-105; Berardesca E. et al., 1990, Acta Derm. Venereol., 70: 400-404).

It has been shown that, in subjects with irritable skin, the TEWL is increased in comparison with normally responsive subjects. It is also known that this parameter increases further as a result of exposure of the irritable skin to hyper-reactive stimuli and that this phenomenon is frequently associated with the development of superficial skin reactions which alter epidermal appearance (Di Nardo A. et al., 1998, Acta Derm. Venereol., 78: 27 -30). It is also known that, in these conditions, a mechanical rubbing action, or the superimposition of several stimuli such as, for example, the hyper-reactive amplification produced by contact with allergenic substances used in daily hygiene, both in man and in animals, bring about progression towards epidermal lesions which are easily infected.

Even in normal conditions, irritable skin has clear unsightly effects which are generally recognizable in the form of dryness (Di Nardo A. et al., 1998, Acta Derm. Venereol., 78: 27-30) or of thinning of the skin which easily predispose to the development of loss of epithelium.

Irritable skin has therefore been defined as the epidermal functional state which characterizes a population of predominantly healthy subjects in certain paraphysiological situations (ageing, pregnancy, menopausal states, pre-menstrual and post-menstrual situations, etc.).

Irritable skin and/or mucous membrane is a tissue which is easily predisposed to hyper-reactive symptoms in response to a diversified set of exogenous stimuli. As a result of stimuli of a neurogenic, immunogenic, physical (for example, radiation), chemical (for example, detergents, solvents, dyes, etc.), mechanical and/or traumatic nature, irritable skin easily develops clear signs of irritation with consequent unsightly effects (Ansel J.C., 1996, J. Invest. Dermatol., 106: 198-204).

It is also known that, precisely because of the altered barrier function and of the altered proliferative and differentiational process, whether it be epidermal or mucosal, irritable skin is predisposed to the development and progression of microbial infections both fungal and bacterial (Leibovici V. et al., 1995, Clin, Exp. Dermatol., 20: 390-394). It is also known that the altered cutaneous and mucosal barrier function explains a large proportion of recurrent and relapsing hyper-reactive episodes in the course of - or as a result of - microbial infections with particular reference to the palmar and plantar skin locations as well as the vulvar and preputial locations.

In the presence of irritable skin and/or mucous membrane, the identification of molecules which reestablish the TEWL values which are functional in keeping the cutaneous and/or mucosal barrier in good condition, offering a modification and a protection from unsightly effects associated with irritable cutaneous and/or mucosal states, is therefore of considerable interest for the production of preparations for cosmetic use which are free of potential hyper-reactive stimulation effects and consequently do not result in adverse reactions due to contact.

The patent publication US 5,618,842 discloses in general terms the dermocosmetic use of amide compounds, which also encompass adelmidrol.

The patent publication US 5,693,623 reports the use of adelmidrol for the treatment of Acne juvenilis, Acne vulgaris and related pathologies.

None of such prior art documents discloses the activity of adelmidrol in improving an altered hydration state of skin tissues.

It has surprisingly been found that adelmidrol (N,N¹-bis(2-hydroxyethyl)nonandiamide) can lower abnormally raised TEWL values in subjects (human or animal) with skin and/or mucous membranes which are irritable and/or subject to acute irritation. It is therefore clear that adelmidrol can advantageously be used as a cosmetic agent or additive for the preparation of cosmetic compositions suitable for subjects with skin and/or mucous membranes which are irritable and/or subject to acute irritation.

The cosmetic effectiveness of adelmidrol is demonstrated by the following example.

### EXAMPLE A

TEWL was measured with the use of atmometer model EPl (ServoMed - Sweden) which is based on estimation of the water-vapour gradient. All of the TEWL measurements were taken in accordance with the method described in Pinnagoda J. et al., 1990, Contact Dermatitis, 22: 164-178.

The measurements were taken on two groups of volunteer subjects, divided into:
- n=10 subjects aged between 25 and 29 years, who were normally reactive from a cutaneous point of view, without previous medical history of adverse reactions due to contact, or of atopia;
- n=10 subjects aged between 65 and 70 years, without previous medical history of dermatitis due to contact, or of atopia.

The areas of topical application of the experimental preparations and of TEWL measurement corresponded to circular areas of skin approximately 2 cm in diameter located in the medial volar region of the right forearm and spaced at least 2 cm apart. The corresponding areas of skin of the medial volar region of the left forearm were used as non-treated controls, upon each analysis.

The various preparations were applied in accordance with the following scheme:
(a) excipients alone (Example 1 of cosmetic preparations)
(b) complete preparation (Example 1 of cosmetic preparations containing 2% of adelmidrol)
(c) sodium lauryl sulphate (SLS) 5% as irritant
(d) 5% SLS + (a)
(e) 5% SLS + (b)

Preparations (a) - (e) were applied for 10 consecutive days. After application, each individual area treated was covered with a plaster. The TEWL measurements were taken on day 1 and on day 10, 1 hour after topical application of the preparations, after the plaster had been removed and the area had been cleaned with absorbent paper.

The data, expressed as Mean ± SEM are summarized in the following table.

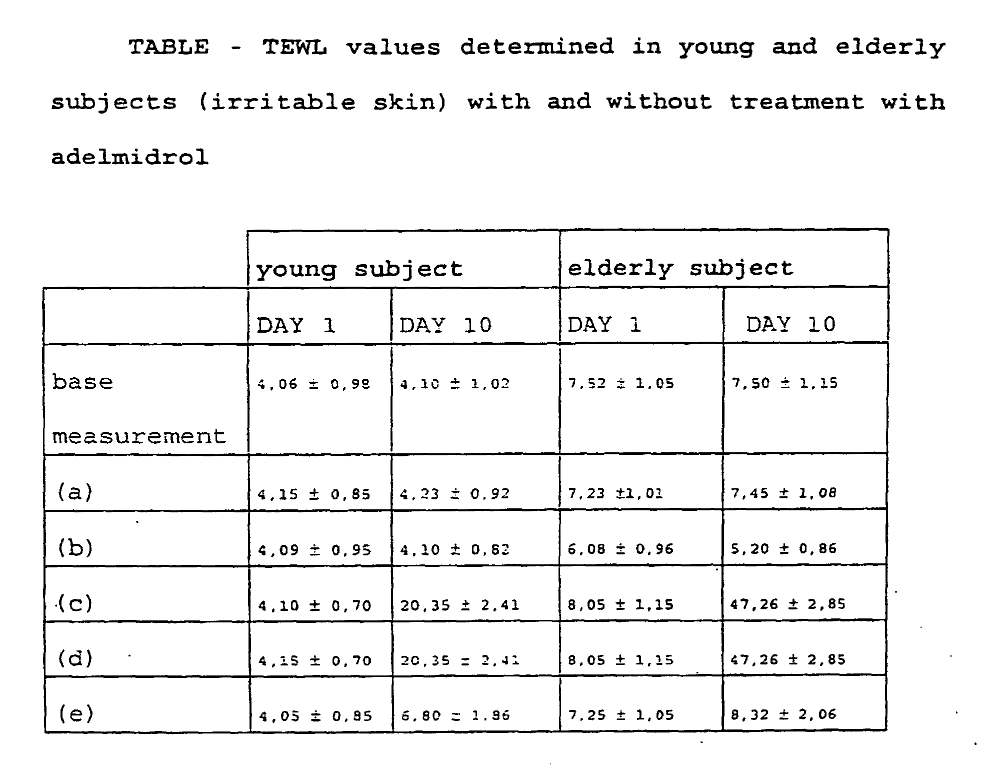

It is clear from the data shown in the table that adelmidrol can practically normalize TEWL values in subjects with irritable skin (elderly patients), bringing the TEWL from a base value of 7.50 (± 1.15) which is abnormally high, to a value of 5.20 (± 0.86) which is close to normality.

Moreover, adelmidrol brought about a clear reduction in TEWL values in both patients with normal skin (young patients) and patients with irritable skin (elderly patients), whose skin had been irritated acutely by contact with 5% SLS.

As stated above, a reduction in TEWL values enables the unsightly effects associated with an irritated and/or irritable skin (dry skin, reddening) to be resisted. Adelmidrol can consequently be used advantageously for the preparation of cosmetic compositions in the treatment of skin and/or mucous membranes which are irritable and/or are subject to acute irritation by lowering abnormally raised TEWL values.

### EXAMPLES OF COSMETIC PREPARATIONS

In all of the following examples, the initials OE mean "oxyethylenate".

### Example 1 - Face and body cream

| 100 g contains: | |
|---|---|
| N,N¹-bis(2-hydroxyethyl)nonandiamide | 2.0 g |
| Vitamin E acetate | 4.0 g |
| sodium hyaluronate | 0.04 g |
| bronopol | 0.005 g |
| hydrogenated castor oil 40 (OE) | 1.5 g |
| noveon AA1 | 1.6 g |
| o-phenylphenol | 0.18 g |
| aroma | 0.15 g |
| water to make up to 100 g | |

### Example 2 - body milk

| 100 g contains: | |
|---|---|
| N,N¹-bis(2-hydroxyethyl)nonandiamide | 1.4 g |
| glycerol | 5.0 g |
| Vaseline oil | 3.0 g |
| silicone oil | 1.0 g |
| gliceryl monostearate | 1.4 g |
| cetostearyl alcohol | 2.8 g |
| stearic acid | 2.8 g |
| polyethylene glycol-soya sterols | 6.0 g |
| carbomer | 0.12 g |
| bronopol | 0.05 g |
| aroma | 0.05 g |
| water to make up to 100 g | |

### Example 3 - gel for oral use

| 100 g contains: | |
|---|---|
| N,N¹-bis(2-hydroxyethyl)nonandiamide | 1.0 g |
| glycerol | 10.0 g |
| glycolic extract of echinacea purpurea | 12.0 g |
| sodium alginate | 2.5 g |
| sodium hyaluronate | 0.04 g |
| bronopol | 0.1 g |
| triclosan | 0.3 g |
| water to make up to 100 g | |

### Example 4 - lotion for trichological use

| 100 g contains: | |
|---|---|
| N,N¹-bis(2-hydroxyethyl)nonandiamide | 0,2 g |
| sodium hyaluronate | 0,01 g |
| biotin | 0,03 g |
| ethyl alcohol | 30,0 g |
| aroma | 0,02 g |
| water to make up to 100 g | |

### Example 5 - vaginal gel

| 100 g contains: | |
|---|---|
| N,N¹-bis(2-hydroxyethyl)nonandiamide | 1.5 g |
| vitamin A palmitate | 0.2 g |
| 2-phenyl ethanol | 0.15 g |
| glycerol | 10.0 g |
| hydrogenated castor oil 40 (OE) | 1.0 g |
| methyl paraoxybenzoate | 0.1 g |
| noveon AA1 | 1.0 g |
| sodium hyaluronate | 0.08 g |
| aroma | 0.2 g |
| water to make up to 100 g | |

### Example 6 - vaginal wash

| 100 g contains: | |
|---|---|
| N,N¹-bis(2-hydroxyethyl)nonandiamide | 0.4 g |
| vitamin A palmitate | 0.02 g |
| 2-phenyl ethanol | 0.15 g |
| lactic acid | 2.0 g |
| glycerol | 6.0 g |
| hydrogenated castor oil 40 (OE) | 0.4 g |
| methyl paraoxyibenzoate | 0.1 g |
| sodium hyaluronate | 0.01 g |
| aroma | 0.15 g |
| water to make up to 100 g | |

### Example 7 - intimate soap

| 100g contains: | |
|---|---|
| N,N¹-bis(2-hydroxyethyl)nonandiamide | 0.5 g |
| triclosan | 0.25 g |
| vitamin E acetate | 0.05 g |
| lactic acid | 1.0 g |
| rutin | 0.02 g |
| bronopol | 0.1 g |
| coco diethanolamide | 2.5 g |
| undecylenic acid | 0.2 g |
| glycolic extract of Echinacea purpurea | 5.0 g |
| sodium lauryl (1-4)OE sulphate | 20.0 g |
| disodium lauryl (1-4)OE sulphosuccinate | 7.0 g |
| aroma | 0.02 g |
| hyrogenated castor oil 40 (OE) | 1.4 g |
| water to make up to 100 g | |

### Example 8 - deodorant stick

| 100 g contains: | |
|---|---|
| N,N¹-bis(2-hydroxyethyl)nonandiamide | 2.0 g |
| sodium hyaluronate | 0.05 g |
| zinc ricinoleate | 1.0 g |
| triclosan | 0.25 g |
| vitamin E acetate | 0.5 g |
| ethyl alcohol | 30.0 g |
| bronopol | 0.05 g |
| carbomer | 1.5 g |
| polyvinyl alcohol | 0.2 g |
| hydrogenated castor oil 40 (OE) | 2.5 g |
| aroma | 1.0 g |
| water to make up to 100 g | |

### Example 9 - nail drops

| 100 g contains: | |
|---|---|
| N,N¹-bis(2-hydroxyethyl)nonandiamide | 1.5 |
| urea | 10.0 |
| hydrogenated castor oil 40 (OE) | 5.0 |
| vitamin A palmitate | 0.3 |
| undecylenic acid | 0.2 |
| biotin | 0.6 |
| benzyl alcohol | 1.2 |
| glycolic extract of | |
| Echinacea purpurea | 10.0 |
| acetone | 8.0 |
| isopropyl alcohol | 36.0 |
| water to make up to 100 g | |

### Example 10 - cream for podiatric use

| 100 g contains: | |
|---|---|
| N,N¹-bis(2-hydroxyethyl)nonandiamide | 2.0 |
| biotin | 0.01 |
| glycolic extract of | |
| Echinacea purpurea | 10.0 |
| usnic acid | 0.2 |
| zinc ricinoleate | 2.0 |
| isopropyl myristate | 0.5 |
| polyethylene glycol-soya sterols | 4.0 |
| stearic acid | 2.5 |
| cetostearyl alcohol | 2.5 |
| glyceryl monostearate | 1.0 |
| silicone oil | 1.0 |
| Vaseline oil | 2.5 |
| triclosan | 0.2 |
| bronopol | 0.03 |
| aroma | 0.5 |
| water to make up to 100 g | |

It has also been found that the cosmetic effect of adelmidrol is performed especially if the product has a high degree of purity. In particular, an absence of potentially harmful impurities prevents the compound itself from causing undesired irritative reactions on the skin. A further subject of the present invention is therefore a method which enables extremely pure adelmidrol to be produced.

This method provides for the reaction of azelaic acid or a diester thereof with ethanolamine, with refluxing, in an inert atmosphere, possibly in the presence of an inert solvent. Preferred solvents for the azelaic acid reaction are xylene or toluene, particularly xylene. Preferred reaction temperatures are between 110°C and 145°C. Dimethyl, diethyl or dipropyl esters, preferably dimethyl ester, may be used as the diester of azelaic acid.

The method may also provide for a step for crystallization of the crude product from isopropanol, in which the crystallizate is subjected to a crumbling process at approximately 45°C for 4-8 hours, and to subsequent gradual cooling to 5-7°C over a period of 10-20 hours. This crystallization step enables a particularly pure product to be produced.

A further improvement in the purity of the adelmidrol can be achieved by subjecting the crystallizate to a drying step comprising a first drying stage at a pressure lower than 30 mmHg and at a temperature of between ambient temperature and 40°C for 24-72 hours, a second drying stage under the same conditions as described above but in a slightly acid atmosphere such as that obtained by placing a container containing a dilute solution of sulphuric acid in the dryer, and a third drying stage at a pressure of 1-2 mmHg and at a temperature of 25-40°C for a period of between 1 and 3 days.

The diester of azelaic acid is prepared with a practically quantitative yield by reaction of azelaic acid with a primary aliphatic alcohol, hot, in the presence of an acid catalyst (Vogel, Chimica Organica Practica, Ed. Casa Editrice Ambrosiana, Milan 1988). The preferred alcohols are methyl, ethyl and propyl alcohols, most preferably methyl alcohol.

Two examples of preparations in accordance with the method of the present invention are given by way of indication below.

### Preparation 1 - synthesis of adelmidrol from azelaic acid

47.05 g of azelaic acid (250 mmoles) and 45.8 g of ethanolamine (750 mmoles) were dissolved in 130 ml of xylene and heated with refluxing for 6 hours in a reactor with a reflux condenser and a Dean-Stark separator. The atmosphere in the reactor was rendered inert with nitrogen at atmospheric pressure. The mixture was then evaporated to dryness under vacuum. The residue was dissolved in 250 ml of pure isopropanol, the solution was then cooled to trigger crystallization, the crystallizate was then subjected to a crumbling process with stirring at 45°C for 6 hours. The crystallization was completed by subsequent slow cooling to 5-7°C over a period of 15 hours. The crystallizate was separated by filtration, cold, in an inert atmosphere, washed three times with 30 ml of cold isopropanol and finally dried in a high degree of vacuum.

The drying was performed in stages as described below. The crystallizate, taken up in solvent, was stratified in polished stainless-steel trays and placed in a drying cabinet for static drying for two days at 30°C and at a pressure lower than 30 mmHg. Trays containing an approximately 1 M solution of sulphuric acid in water were then placed in the drying cabinet for 24 hours, again with a pressure lower than 30 mmHg. Drying was then completed under a vacuum of 1-2 mmHg for 2 days at 30°C.

The reaction yields were greater than 96%, with a recovery of more than 90% of pure product after crystallization and drying.

The profile of the impurities of the finished product was as follows:

| | |
|---|---|
| free azelaic acid | <0.5% |
| monoethanolamide | <0.5% |
| free ethanolamine | <0.1% |
| isopropanol | <200 ppm |
| homologous diamides | 1-10%(*) |
| 2-oxazoline | absent |

| | |
|---|---|
| (*) according to the quality of the starting azelaic acid | |

### Preparation 2 - synthesis of adelmidrol from azelaic acid dimethyl ester

54.1 g of the dimethyl ester of azelaic acid (250 mmoles) and 33.6 g of ethanolamine (550 mmoles) were heated to 110°C for 7 hours in a reactor with a reflux condenser. The atmosphere in the reactor was rendered inert by nitrogen at atmospheric pressure. The mixture was then evaporated to dryness under vacuum. The residue was dissolved in 250ml of pure isopropanol, the solution was then cooled to trigger crystallization and the crystallizate was subjected to a crumbling process with stirring at 45°C for 6 hours. The process was completed by subsequent slow cooling to 5-7°C over a period of 15 hours. The crystallizate was separated by filtration, cold, in an inert atmosphere, washed three times with 30 ml of cold isopropanol and finally dried in a high degree of vacuum.

The drying was performed in stages as described below. The crystallizate, taken up with solvent, was stratified in polished stainless-steel trays and placed in a drying cabinet for static drying for two days at 30°C and at a pressure lower than 30 mmHg. Trays containing an approximately 1M solution of sulphuric acid in water were then placed in the drying cabinet for 24 hours, again with a pressure lower than 30 mmHg. Drying was then completed under a vacuum of 1-2 mmHg for 2 days at 30°C.

The reaction yields were again greater than 96%, with a recovery of more than 90% of pure product after crystallization and drying.

The profile of the impurities of the finished product was as follows:

| | |
|---|---|
| free azelaic acid | <0.1% |
| monoethanolamide | <0.2% |
| free ethanolamine | <0.1% |
| isopropanol | <200 ppm |
| homologous diamides | <1% |
| 2-oxazoline | absent |

The method described above thus has the advantage of leading to a final product which is characterized by a high degree of purity, with higher yields than can be obtained by known methods (for example, see EP 0 550 0008 A2) and with lower running costs by virtue of the lower reaction temperatures (no greater than 145°C in comparison with 160°C described in the known methods) . The reaction starting with the diester of azelaic acid has the further advantage of permitting purification even at the level of the starting product by fractional distillation of the diester. The lower and higher homologues of azelaic acid which are always present in considerable quantities in commercial azelaic acid (titre between 80 and 90%) can thus be eliminated.

## Claims

1. Non-therapeutic use of N,N¹-bis(2-hydroxyethyl)nonandiamide, in a cosmetic composition, for lowering abnormally raised TEWL values in skin and/or mucous membranes which are irritable and/or are subject to acute irritation.

2. Use according to Claim 1, for the cosmetic treatment of the skin of the face, of the body, of the hands, of the feet, of the genital organs, and of the mucous membrane of the mouth, of the vagina and of the glans.

3. A method of preparing N,N¹-bis(2-hydroxyethyl)nonandiamide comprising the reaction of azelaic acid or of a diester thereof with ethanolamine in an inert atmosphere, possibly in the presence of an inert solvent.

4. A method according to Claim 3, in which the diester of azelaic acid is dimethyl, diethyl or dipropyl ester, preferably dimethyl ester.

5. A method according to Claim 3 or Claim 4, in which the reaction temperature is between 110°C and 145°C.

6. A method according to any one of Claims 3 to 5 in which the solvent is xylene.

7. A method according any one of Claims 3 to 6, comprising the further step of crystallizing the crude product from isopropanol and subjecting the crystallizate to a crumbling process at approximately 45°C for 4-8 hours and to subsequent gradual cooling to 5-7°C over a period of 10-20 hours.

8. A method according to any one of Calims 3 to 7, comprising the further consecutive steps of:
(i) drying N,N¹-bis(2-hydroxyethyl)nonandiamide at a pressure lower than 30 mmHg and at a temperature of between ambient temperature and 40°C for 24-72 hours,
(ii) further drying the product of stage (i) under the same conditions as described above but in a slightly acid atmosphere,
(iii) further drying the product of stage (ii) at a pressure of 1-2 mmHg and at a temperature of 25-40°C for a period of between 1 and 3 days.

9. A method according to Claim 8, in which the acid atmosphere in stage (ii) is achieved by introducing a container containing a 1M solution of sulphuric acid into the dryer.

10. Use according to Claim 1 or Claim 2, wherein said N,N¹-bis(2-hydroxyethyl)nonandiamid is as obtainable by the method of any ony of claims 3 to 9.

## Patentansprüche

1. Nichttherapeutische Verwendung von N,N¹-Bis(2-hydroxyethyl)nonandiamid in einer kosmetischen Zusammensetzung zum Senken anomal erhöhter TEWL-Werte in Haut und/oder Schleimhäuten, die reizbar sind und/oder akuter Reizung ausgesetzt sind.

2. Verwendung nach Anspruch 1 zur kosmetischen Behandlung der Haut des Gesichts, des Körpers, der Hände, der Füße, der Geschlechtsorgane und der Mundschleimhaut, der Vaginalschleimhaut und der Schleimhaut der Eichel.

3. Verfahren zur Herstellung von N,N¹-Bis(2-hydroxyethyl)nonandiamid, umfassend die Umsetzung von Azelainsäure oder einem Diester davon mit Ethanolamin in einer Inertatmosphäre, gegebenenfalls in Gegenwart eines inerten Lösungsmittels.

4. Verfahren nach Anspruch 3, wobei der Azelainsäurediester der Dimethyl-, Diethyl- oder Dipropylester, vorzugsweise der Dimethylester ist.

5. Verfahren nach Anspruch 3 oder Anspruch 4, wobei die Reaktionstemperatur zwischen 110°C und 145°C liegt.

6. Verfahren nach irgendeinem der Ansprüche 3 bis 5, wobei das Lösungsmittel Xylol ist.

7. Verfahren nach irgendeinem der Ansprüche 3 bis 6, welches den weiteren Schritt umfaßt, daß das Rohprodukt aus Isopropanol kristallisiert wird und das Kristallisat einem 4-8-stündigen Zerkrümelungsprozeß bei etwa 45°C und anschließend für eine Dauer von 10-20 Stunden einer allmählichen Abkühlung auf 5-7°C unterworfen wird.

8. Verfahren nach irgendeinem der Ansprüche 3 bis 7, welches die weiteren aufeinanderfolgenden Schritte umfaßt:
(i) Trocknen von N,N¹-Bis(2-hydroxyethyl)nonandiamid über 24-72 Stunden bei einem Druck unter 30 mmHg und bei einer Temperatur zwischen Raumtemperatur und 40°C,
(ii) Weitertrocknen des Produkts aus Stufe (i) unter den gleichen Bedingungen wie oben beschrieben, jedoch in einer schwach sauren Atmosphäre,
(iii) Weitertrocknen des Produkts aus Stufe (ii) bei einem Druck von 1-2 mmHg und bei einer Temperatur von 25-40°C für eine Dauer von 1 bis 3 Tagen.

9. Verfahren nach Anspruch 8, wobei die saure Atmosphäre in Stufe (ii) erreicht wird, indem man in die Trockenvorrichtung einen Behälter einbringt, der eine 1M Schwefelsäurelösung enthält.

10. Verwendung nach Anspruch 1 oder Anspruch 2, wobei das N,N¹-Bis(2-hydroxyethyl)nonandiamid ein solches ist, wie es nach dem Verfahren nach irgendeinem der Ansprüche 3 bis 9 erhältlich ist.

## Revendications

1. Utilisation non thérapeutique du N,N¹-bis(2-hydroxyéthyl)nonanediamide, dans une composition cosmétique, destinée à diminuer les valeurs de PIE anormalement élevées de la peau et/ou des muqueuses qui sont irritables et/ou sujets à l'irritation aiguë.

2. Utilisation selon la revendication 1, destinée au traitement cosmétique de la peau du visage, du corps, des mains, des pieds, des organes génitaux, et de la muqueuse de la bouche, du vagin et du gland.

3. Procédé de préparation du N,N¹-bis(2-hydroxyéthyl)nonanediamide comprenant la réaction de l'acide azélaïque, ou d'un diester de cet acide, avec de l'éthanolamine dans une atmosphère inerte, en présence possible d'un solvant inerte.

4. Procédé selon la revendication 3, dans lequel le diester de l'acide azélaïque est un ester diméthylique, un ester diéthylique ou un ester dipropylique, de préférence un ester diméthylique.

5. Procédé selon la revendication 3 ou la revendication 4, dans lequel la température de réaction se situe entre 110°C et 145°C.

6. Procédé selon l'une quelconque des revendications 3 à 5, dans lequel le solvant est du xylène.

7. Procédé selon l'une quelconque des revendications 3 à 6, comprenant l'étape supplémentaire consistant à cristalliser le produit brut à partir d'isopropanol et à soumettre le produit cristallisé à un procédé d'effritement à approximativement 45°C pendant 4 à 8 heures, et au refroidissement progressif de 5 - 7°C pendant une période de 10 - 20 heures.

8. Procédé selon l'une quelconque des revendications 3 à 7, comprenant les étapes consécutives supplémentaires consistant à :
(i) sécher le N,N¹-bis(2-hydroxyéthyl)nonanediamide à une pression inférieure à 30 mmHg et à une température située entre la température ambiante et 40°C, pendant 24 - 72 heures,
(ii) sécher davantage le produit de l'étape (i) dans les mêmes conditions comme celles décrites ci-dessus, mais dans une atmosphère légèrement acide,
(iii) sécher davantage le produit de l'étape (ii) à une pression de 1 - 2 mmHg et à une température de 25 - 40°C, pendant une période entre 1 et 3 jours.

9. Procédé selon la revendication 8, dans lequel l'atmosphère acide dans l'étape (ii) est réalisée par l'introduction, dans le dessiccateur, d'un récipient contenant une solution d'acide sulfurique à 1 M.

10. Utilisation selon la revendication 1 ou la revendication 2, dans lequel ledit N,N¹-bis(2-hydroxyéthyl)nonanediamide est peut être obtenu par le procédé de l'une quelconque des revendications 3 à 9.
